Europäisches Patentamt

European Patent Office

Office européen des brevets

(11) Publication number: **0 186 399**
**A2**

# EUROPEAN PATENT APPLICATION

(21) Application number: **85309101.5**

(22) Date of filing: **13.12.85**

(51) Int. Cl.⁴: **C07C 11/06** , C07C 5/333 , B01J 29/30

(30) Priority: **28.12.84 US 686961**
**15.10.85 US 787544**

(43) Date of publication of application:
**02.07.86 Bulletin 86/27**

(84) Designated Contracting States:
**BE DE FR GB IT NL**

(71) Applicant: **MOBIL OIL CORPORATION**
**150 East 42nd Street**
**New York New York 10017(US)**

(72) Inventor: **Page (nee Forbis), Nancy Marie**
**289 Flint Court**
**Yardley Pennsylvania 19067(US)**

(74) Representative: **West, Alan Harry**
**Mobil Court 3 Clements Inn**
**London WC2A 2EB(GB)**

(54) A method and catalyst for converting propane to propylene.

(57) Propane is converted to propylene by dehydrogenation over a catalyst comprising a zeolite, such as ZSM-12, ZSM-35, zeolite Y and mordenite, platinum and optionally an oxide of magnesium and/or manganese.

EP 0 186 399 A2

## A METHOD AND CATALYST FOR CONVERTING PROPANE TO PROPYLENE

This invention relates to a method and catalyst for the conversion of propane to propylene.

Rosinski et al U.S. Patent No. 3,832,449 describes the zeolite, ZSM-12 and indicates that ZSM-12 has ion exchange capacity with a wide variety of metal cations including manganese, calcium and other metals of Group II of the Periodic Table. The Rosinski et al patent also indicates that ZSM-12 can be combined with certain metals such as platinum for hydrogenation-dehydrogenation reactions.

Brennan et al U.S. Patent No. 3,759,821 indicates that optional hydrogenation/dehydrogenation components for incorporation with the zeolites described therein, e.g., ZSM-5, include metals, oxides and sulfides of a wide variety of metals including manganese.

Published European Patent Application No. 0 105 591 describes the conversion of methanol to light olefins especially enriched in propylene using a ZSM-12 catalyst modified with magnesium and/or manganese.

According to one aspect of the invention, there is provided a process for converting propane to propylene, said processs comprising contacting the propane under dehydrogenation conditions with a catalyst comprising platinum and a zeolite capable of sorbing propane.

In a further aspect, the invention resides in a catalyst for use in the process of said one aspect comprising ZSM-12, platinum and an oxide of magnesium and/or manganese.

The zeolites suitable for use in the process of the present invention are those which are capable of sorbing (e.g., at least 2 percent by weight) of propane. Such zeolites are well known. Examples of such zeolites are provided in the Forbus et al U.S. Patent No. 4,504,690. Included within the zeolites capable of sorbing propane are those having a Constraint Index of 12 or less, the term "Constraint Index" and a method of its measurement being disclosed in, for example, U.S. Patent No. 4,016,218. Examples of zeolites with a Constraint Index of 12 or less include:

| Zeolite | C.I. |
|---|---|
| ZSM-5 | 8.3 |
| ZSM-11 | 8.7 |
| ZSM-12 | 2 |
| ZSM-23 | 9.1 |
| ZSM-35 | 4.5 |
| ZSM-38 | 2 |
| ZSM-48 | 3.4 |
| TMA Offretite | 3.7 |
| Clinoptilolite | 3.4 |
| Beta | 1.5 |
| H-Zeolon (mordenite) | 0.4 |
| REY | 0.4 |

Preferably, the zeolite is ZSM-12 (U.S. Patent No. 3,832,449), ZSM-35 (U.S. Patent No. 4,016,245), zeolite Y (particularly the hydrogen form thereof) and mordenite, with ZSM-12 being the most preferred.

Where the zeolite employed is ZSM-12, the latter preferably has a crystal size of 0.02 to 0.5 micron and an acid activity measured in terms of its alpha value of between 25 and 250, e.g., more preferably between 50 and 150. In this respect, measurement of alpha value is disclosed in, for example, the Journal of Catalysis, Vol. VI, Pages 278-287, 1966.

In practicing the propane conversion process of the present invention, it may be useful to incorporate the above-described crystalline zeolites with a matrix comprising another material resistant to the temperature and other conditions employed in the process. Such matrix material is useful as a binder and imparts greater resistance to the catalyst for the temperature, pressure and reactant feed stream velocity conditions which may be encountered in the propane conversion process.

Useful matrix materials include both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix, on an anhydrous basis, may vary widely with the zeolite content ranging from between 1 to 99 percent by weight and more usually in the range of 5 to 80 percent by weight of the dry composite.

The zeolite catalysts employed herein are modified by incorporating thereon a minor proportion of platinum and preferably a minor proportion of magnesium and/or manganese. Such modified zeolite composites can be prepared by contacting the zeolite composition with one or more compounds or complexes of the modifying element(s) and by preferably thereafter heating the catalyst composite to convert the modifying elements to their elemental or oxide form. Incorporation can occur by the mechanisms of ion exchange, adsorption and/or impregnation, the latter two phenomena commonly being referred to as "stuffing." It should be emphasized that, while ion exchange can be used to incorporate the modifying metals onto the zeolite compositions described herein, ion exchange alone will generally not provide the requisite amount or form preferred (i.e. the oxide form) of incorporated magnesium and manganese onto the zeolite catalyst composites used in the present invention. More particularly, the amount of magnesium and/or manganese plus platinum may exceed the amount capable of occupying all of the cation exchange sites of the zeolite by a factor of, e.g., 2 or more or even 5 or more.

Generally, the zeolite composites used in the present invention can be modified by contacting such composites with solutions of compounds of the modifying element(s) to be incorporated. Such solutions may be formulated from any suitable solvent which is inert with respect to the metal-containing compound and the zeolite composition. Nonlimiting examples of some suitable solvents include water, aromatic and aliphatic hydrocarbons, alcohols, and organic acids (such as acetic acid, formic acid, propionic acid and so forth). Other commonly available solvents such as halogenated hydrocarbons, ketones and ethers may also be useful to dissolve some magnesium or manganese compounds or complexes. Generally, the most useful solvent will be found to be water. However, the solvent of choice for any particular compound will, of course, be determined by the nature of that compound, and for that reason the foregoing list should not be considered exhaustive of all of the suitable possibilities.

Representative platinum compounds used to modify the zeolites employed here include chloroplatinic acid, platinous chloride and various compounds containing the platinum ammine complex.

Representative magnesium-containing compounds include magnesium acetate, magnesium nitrate, magnesium benzoate, magnesium propionate, magnesium 2-ethylhexanoate, magnesium carbonate, magnesium formate, magnesium oxylate, magnesium amide, magnesium bromide, magnesium hydride, magnesium lactate, magnesium laurate, magnesium oleate, magnesium palmitate, magnesium salicylate, magnesium stearate and magnesium sulfide.

Representative manganese-containing compounds include manganese acetate, manganese nitrate, manganese lactate, manganese oxalate, manganese carbonate, manganese citrate, manganese tartarate, manganese bromide, manganese chloride, manganese sulfate, and manganese sulfide.

The amount of modifying metal incorporated onto the zeolite composition by reaction with these metal-containing compounds will depend upon several factors. One of these is the reaction time, i.e., the time that the zeolite composition and the metal-containing source are maintained in contact with each other. With greater reaction times, all other factors being equal, a greater amount of metal is incorporated with the zeolite. Other factors upon which the amount of modifiers incorporated with the zeolite is dependent include reaction temperature, concentration of the treating compound in the reaction mixture, the degree to which the zeolite composition has been dried prior to reaction with the metal-containing compounds, the conditions of drying of the zeolite composition after reaction with the treating compounds, and the amount and type of binder incorporated with the zeolite composition.

After modifying metals have been incorporated into the zeolite composite to the extent desired, the metal containing composite can be heated subsequent to metal modification and prior to use. Such heating can be carried out in the presence of oxygen, for example, in air. Although heating may be carried out at a temperature of about 150°C, higher temperatures, e.g., up to about 500°C, are preferred. Heating is generally carried out for 1-5 hours but may be extended to 24 hours or longer. While heating temperatures above about 500°C may be employed, they are generally not necessary.

The zeolite composites herein are treated with the foregoing compounds and subsequently heated, under conditions and for a time sufficient to incorporate a minor proportion of each of the modifying elements onto the zeolite composite. Generally, the platinum and any other modifying element(s) are incorporated to the total extent of at least 0.1%, preferably from 0.1% to 10% by weight of the zeolite composite, calculated on the basis of the elemental metals. Preferably, the platinum content is 0.1-2%, the magnesium content is 0.1% to 5% and the manganese content is 1% to 10%, all the percentages being by weight of the zeolite composite and being calculated on the basis of elemental metal.

The resultant modified catalysts are capable of exhibiting a high degree of selectivity when used to convert propane to propylene. For example, products of such a propane conversion may contain 75 percent by weight of propylene. This high degree of propylene selectivity may be maintained even at propane conversion rates as high as, e.g., 20 percent or more. In other terms, all things otherwise being equal, the modified catalyst may increase the weight percent of propylene in the conversion products by a factor of, e.g., at least 2.5 times in comparison with an unmodified catalyst at the same or even higher conversion rates for the modified catalyst, wherein the unmodified catalyst has essentially all of the zeolite in the hydrogen form (e.g., HZSM-12).

Propane may be converted to propylene with the modified catalyst under sufficient dehydrogenation conditions. These conditions may include, for example, a temperature of from 300 to 700°C, a pressure of from 10 to 1013 kPa (0.1 to about 10 atmospheres) and a weight hourly space velocity of propane of from 0.1 to 20.

The invention will now be more particularly described with reference to the following Examples and Comparative Examples.

COMPARATIVE EXAMPLE A

5.0 gm of an HZSM-I2 extrudate (65% zeolite/35% aluminum binder) was placed in a quartz microreactor. Propane was passed over the catalyst at the rate of 40 cc/min. Reactor temperature was maintained at 550°C. The catalyst was then calcined at 538°C for at least an hour. Screening for propane conversion was then repeated at 600°C. Reactor effluent composition was determined by gas chromatography using a silica gel column for light gas separation and an OV-I0I column for a total reactor effluent analysis. Product selectivities and propane conversions were as follows:

| Temperature, °C | 550° | 600° |
|---|---|---|
| Products, wt. % | | |
| $H_2$ | 1.6 | 3.1 |
| $CH_4$ | 29.3 | 27.1 |
| $C_2H_6$ | 10.1 | 7.0 |
| $C_2H_4$ | 32.4 | 31.5 |
| $C_3H_6$ | 16.5 | 28.6 |
| $C_4H_{10}$ | 4.9 | 1.8 |
| $C_4H_8$ | 5.3 | 0 |
| Bz | 0 | 0.7 |
| Tol | 0 | 0 |
| $C_8A$ | 0 | 0 |
| $C_9A$ | 0 | 0 |
| $C_{10}^+$ | 0 | 0 |
| Propane Conversion | 14.2 | 18.4 |

EXAMPLE 1

A catalyst was prepared by adding 10 ml of a solution containing deionized water, 0.7 gm of a I0% solution of $H_2PtCl_4 \cdot 6H_2O$, and I.6 gm of a 50% solution of $Mn(NO_3)_2$ to 5 gm of the ZSM-I2 extrudate used in Comparative Example A. Water was permitted to evaporate slowly (e.g., overnight). The resulting catalyst was calculated to contain 5% Mn and 0.5% Pt by weight. After drying at 75°C, the catalyst was calcined at 500°C in a muffle furnace 5.0 gm of the resulting catalyst was loaded into a quartz microreactor, reduced under $H_2$ at 500°C, then screened for activity for propane conversion at 550° and 600°C at a propane WHSV = 0.9 hr. $^{-1}$. Product selectivities and propane conversions were as follows:

| Temperature, °C | 550° | 600° |
|---|---|---|
| Products, wt. % | | |
| $H_2$ | 5.3 | 5.0 |
| $CH_4$ | 1.6 | 5.5 |
| $C_2H_6$ | 3.1 | 4.4 |
| $C_2H_4$ | 0.2 | 2.7 |
| $C_3H_6$ | 84.0 | 78.9 |
| $C_4H_{10}$ | 0.7 | 0 |
| $C_4H_8$ | 3.1 | 2.2 |
| Bz | 1.8 | 1.0 |
| Tol | 0 | 0 |
| $C_8A$ | 0 | 0 |
| $C_9A$ | 0 | 0 |
| $C_{10}^+$ | 0 | 0 |
| Propane Conversion | 23.8 | 26.8 |

EXAMPLE 2

Another catalyst was prepared by adding 10 ml of a solution containing deionized water, 0.7 gm of a l0% solution ofH₂PtCl₆ • 6H₂O. and 2.6 gm Mg(NO₃)₂ • 6H₂O to 5 gm of the ZSM-l2 extrudate used in Comparative Example A. Water was permitted to evaporate slowly (e.g., overnight). The resulting catalyst was dried at 75°C then calcined at 500°C in a muffle furnace. This catalyst was calculated to contain 5% Mg and 0.5% Pt. by weight. 5.0 gm of this catalyst was loaded into a quartz microreactor, reduced under H₂ at 500°C, then screened for activity in propane conversion at 550° and 600°C at a propane WHSV = 0.9 hr.⁻¹. Product selectivities and propane conversions were as follows:

| Temperature, °C | 550° | 600° |
|---|---|---|
| Products, wt. % | | |
| $H_2$ | 4.6 | 3.9 |
| $CH_4$ | 2.4 | 6.9 |
| $C_2H_6$ | 2.7 | 5.8 |
| $C_2H_4$ | 0.1 | 1.5 |
| $C_3H_6$ | 87.1 | 79.3 |
| $C_4H_{10}$ | 0.5 | 0 |
| $C_4H_8$ | 1.5 | 1.6 |
| Bz | 0 | 0 |
| Tol | 1.1 | 0.8 |
| $C_8A$ | 0 | 0 |
| $C_9A$ | 0 | 0 |
| $C_{10}^+$ | 0 | 0 |
| Propane Conversion | 22.7 | 32.0 |

COMPARATIVE EXAMPLE B

To determine the activity of the Pt-Mg components in the absence of the zeolite component, a catalyst was prepared by adding 5 gm MgO (l2/20 mesh Harshaw magnesia catalyst, MG-060l) to 10 ml of a solution containing deionized water and 0.7 gm of a l0% solution of $H_2PtCl_4$ • $6H_2O$. Water was permitted to evaporate slowly (e.g., overnight).

This catalyst was calculated to contain 0.5% Pt by weight on MgO. After drying at 75°C, the catalyst was calcined at 500°C in a muffle furnace 5.0 gm of the catalyst was loaded into a quartz microreactor, reduced under $H_2$ at 500°C, then screened for activity for propane conversion at 600°C at a propane WHSV = 0.9 hr.[-1].

For comparison, a quartz microreactor was packed with low surface area quartz chips. The reactor was heated to 600°C and propane was passed through the reactor at the same flow rate used during catalyst screening. Product selectivities and propane conversion observed at 600°C for the Pt-MgO catalyst and for the thermal reaction are given below.

| Catalyst | Pt-MgO | None (Quartz Chips) |
|---|---|---|
| Products, Wt. % | | |
| $H_2$ | 4.3 | 2.2 |
| $CH_4$ | 11.9 | 17.3 |
| $C_2H_6$ | 1.2 | 1.7 |
| $C_2H_4$ | 15.2 | 28.9 |
| $C_3H_6$ | 58.6 | 44.7 |
| $C_4H_{10}$ | 6.4 | 5.0 |
| $C_4H_8$ | 0 | 0 |
| Bz | 0.2 | 0 |
| Tol | 0 | 0 |
| $C_8A$ | 0 | 0 |
| $C_9A$ | 0 | 0 |
| $C_{10}+$ | 0 | 0 |
| Propane Conversion | 6.5 | 4.6 |

The foregoing Examples and Comparative Examples illustrate that neither the zeolite component nor the modifying metals alone give the high selectivity to propylene of the modified zeolite catalyst. However, the combination of the zeolite and the modifying metals give a catalyst which is more active for propane conversion and more selective for propylene than either component individually.

EXAMPLE 3

The hydrogen forms of ZSM-35 (HZSM-35), zeolite Y (HY) and mordenite (H-mordenite) were modified with Pt to contain 0.5 wt % Pt by impregnation with $H_2PtCl_4$. Results of propane conversions obtained with both the modified and unmodified catalysts are summarized as follows:

| Catalyst | HZSM-35 | Pt-ZSM-35 | HY | Pt-Y | H-mordenite | Pt-mordenite |
|---|---|---|---|---|---|---|
| Temp., °C | 550 | 550 | 600 | 600 | 550 | 550 |
| $C_3H_8$ Conversion | 18.7 | 22.5 | 24.8 | 21.6 | 10.7 | 13.6 |

Product Selectivities, wt%

| | | | | | | |
|---|---|---|---|---|---|---|
| $H_2$ | 1.7 | 2.8 | 7.6 | 4.8 | 7.1 | 3.1 |
| $CH_4$ | 29.1 | 13.9 | 38.5 | 11.6 | 29.7 | 17.3 |
| $C_2H_6$ | 4.2 | 3.8 | 20.2 | 3.0 | 11.8 | 1.3 |
| $C_2H_4$ | 33.4 | 1.5 | 8.9 | 10.8 | 15.8 | 3.3 |
| $C_3H_6$ | 15.3 | 72.5 | 13.3 | 62.6 | 15.8 | 67.7 |
| $C_4H_{10}$ | 1.7 | 0 | 2.1 | 0.2 | 9.0 | 0.1 |
| $C_4H_8$ | 4.8 | 2.7 | 2.7 | 2.3 | 6.3 | 3.3 |
| Aroms | 9.9 | 2.6 | 6.3 | 4.2 | 3.3 | 2.5 |
| | | | | | | |
| C2=/C3=ratio | 2.18 | 0.02 | 0.67 | 0.17 | 1.0 | 0.05 |
| C2=-C4=select. | 53.5 | 76.7 | 24.9 | 75.7 | 37.9 | 74.3 |

**Claims**

1. A process for converting propane to propylene, said process comprising contacting the propane under dehydrogenation conditions with a catalyst comprising platinum and a zeolite capable of sorbing propane.

2. A process according to Claim 1, wherein said zeolite has a silica/alumina mole ratio of at least 12.

3. A process according to Claim 1 or Claim 2, wherein said zeolite has a Constraint Index of 12 or less.

4. A process according to any preceding claim, wherein said zeolite is selected from ZSM-12, ZSM-35, zeolite Y and mordenite.

5. A process according to any preceding claim, wherein said zeolite is ZSM-12.

6. A process according to any preceding claim, wherein said catalyst comprises at least about 0.1 percent by weight of platinum, calculated on the basis of the elemental metal platinum.

7. A process according to any preceding claim wherein the catalyst also comprises an oxide of magnesium and/or manganese.

8. A process according to any preceding claim wherein the catalyst comprises 0.1 to 2% platinum, 0.1 to 5% of an oxide of magnesium and 1 to 10% by weight of an oxide of manganese, all calculated by weight of the zeolite catalyst on the basis of elemental metal.

9. A process according to any preceding claim, wherein said dehydrogenation conditions comprise a temperature of from 300°C to 700°C, a pressure of from 10 to 1013 kPa (0.1 to 10 atmospheres) and a weight hourly space velocity of propane of from 0.1 to 20.

10. A catalyst for use in the process of Claim 1 comprising ZSM-12, platinum and an oxide of magnesium and/or manganese.

11. A catalyst according to Claim 10 wherein the catalyst comprises 0.1 to 2% platinum, 0.1 to 5% of an oxide of magnesium and 1 to 10% by weight of an oxide of manganese, all calculated by weight of the zeolite catalyst on the basis of elemental metal.